# EUROPÄISCHE PATENTSCHRIFT

(11) **EP 1 098 629 B1**
(45) Veröffentlichungstag und Bekanntmachung des Hinweises auf die Patenterteilung: **25.09.2002**
(21) Anmeldenummer: 99940031.0
(22) Anmeldetag: 21.07.1999
(51) Int. Cl.: A61K 7/16

(54) **ENTZÜNDUNGSHEMMENDE ZAHNPFLEGEMITTEL**
ANTI-INFLAMMATORY DENTAL CARE AGENTS
PRODUITS DE SOINS DENTAIRES A EFFET ANTI-INFLAMMATOIRE

(30) Priorität: 30.07.1998 DE 19834355
(43) Veröffentlichungstag der Anmeldung: 16.05.2001
(73) Patentinhaber: Henkel Kommanditgesellschaft auf Aktien, 40589 Düsseldorf-Holthausen (DE)
(72) Erfinder: LEINEN, Hans-Theo, D-40229 Düsseldorf (DE); WÜLKNITZ, Peter, D-42799 Leichlingen (DE); JASSOY, Claudia, D-40235 Düsseldorf (DE)
(86) Internationale Anmeldenummer: EP9905210
(87) Internationale Veröffentlichungsnummer: WO00006109

(56) Entgegenhaltungen:
- EP-A- 0 353 675
- EP-A- 0 528 468
- GB-A- 1 514 469
- US-A- 5 188 817
- CHEMICAL ABSTRACTS, vol. 116, no. 18, 4. Mai 1992 (1992-05-04) Columbus, Ohio, US; abstract no. 181171, ORBAN, GYULA ET AL: "Roborant oral composition comprising vitamins, amino acids and trace elements" XP002124960 & HU 57 046 A (HUNG.) 28. November 1991 (1991-11-28)
- DATABASE WPI Section Ch, Week 199631 Derwent Publications Ltd., London, GB; Class B05, AN 1996-306469 XP002124961 & JP 08 133969 A (LION CORP), 28. Mai 1996 (1996-05-28)

## Beschreibung

Die Erfindung betrifft Mittel zur Reinigung und Pflege der Zähne, die aufgrund einer speziellen Wirkstoffkombination Entzündungen des Zahnfleisches (Gingivitis) entgegenwirkt.

Zahnpflegemittel dienen in erster Linie der Reinigung der Zahnoberfläche von Speiseresten, Verfärbungen und fest anhaftenden bakteriellen Zahnbelägen. Darüber hinaus wird versucht, durch spezielle Zusätze, z.B. durch Fluorverbindungen oder antimikrobielle Stoffe den Erkrankungen der Zähne und des Zahnfleisches wie z.B. Karies, Gingivitis oder Parodontose entgegenzuwirken.

Eine besonders hartnäckige Erkrankung, die - wenn sie nicht erfolgreich bekämpft wird - zur Lockerung und zum Verlust von Zähnen führen kann, ist die Parodontitis, die im Anfangsstadium durch Entzündung und Blutung des Zahnfleisches (Gingivitis) in Erscheinung tritt. Sie wird durch Bakterien verursacht, die in den Zahntaschen siedeln und durch mechanische Reinigung der Zähne mit der Zahnbürste nur schwer zu bekämpfen sind.

Man hat versucht, durch Zusatz von antibakteriellen Stoffen in Zahnpasten und Mundwässern Zahnfleischentzündungen zu bekämpfen, ein durchschlagender Erfolg ist aber in den meisten Fällen damit nicht zu erzielen. Als besonders wirksam haben sich zwar bestimmte nicht-kationische bakterizide Stoffe, insbesondere aus der Gruppe der chlorierten Diphenylether erwiesen, aber auch diese Stoffe sind allein nicht in der Lage, die hartnäckigen Zahnfleischentzündungen wirksam zu bekämpfen.

Aus der amerikanischen Patentschrift US 5,188,817 ist ein Verfahren zur Behandlung des Periodontalgewebes mit einer pharmazeutischen Zusammensetzung bekannt, die ein Vitamin-A-Derivat enthält. Auch Zahnpasten mit Vitamin A sind seit langer Zeit im Handel (Aronal®).

Es wurde nunmehr festgestellt, daß insbesondere solche Zahnpasten, die bereits eine antibakterielle Komponente enthalten, in ihren entzündungshemmenden Eigenschaften merklich verbessert werden können, wenn man ihnen eine Kombination aus einem Panthenol oder Pantothenat und einem Retinol oder Retinolderivat zusetzt.

Gegenstand der Erfindung ist daher ein Zahnpflegemittel mit entzündungshemmender Wirkung mit einem Gehalt an Poliermitteln, Fluorverbindungen, Feuchthaltemitteln, Bindemitteln, einer wasserunlöslichen, nicht kationischen bakteriziden Komponente und Wasser, die zur Verbesserung der entzündungshemmenden Eigenschaften eine Kombination von Panthenol oder einem Salz der Pantothensäure und Retinol oder ein Derivat davon enthält.

Zahnpflegemittel im Sinne der Erfindung sind Zahnpulver, Zahnpasten, flüssige Zahncremes und Zahngele. Bevorzugt geeignet sind Zahnpasten und flüssige Zahnreinigungsmittel.

Als Poliermittel eignen sich prinzipiell alle für Zahnpasten bekannten Reibkörper, insbesondere solche, die keine Calciumionen enthalten. Bevorzugt geeignete Poliermittelkomponenten sind daher Kieselsäuren, Aluminiumhydroxid, Aluminiumoxid, Natriumaluminiumsilikate, organische Polymere oder Gemische solcher Reibkörper.

Calciumhaltige Polierkomponenten wie z.B. Kreide, Calciumpyrophosphat, Dicalciumphosphat-dihydrat können aber in Mengen bis zu 5 Gew.-% enthalten sein.

Der Gesamtgehalt an Poliermitteln liegt vorzugsweise im Bereich von 5 - 50 Gew.-% des Zahnpflegemittels.

Besonders bevorzugt sind Zahnpasten und flüssige Zahnreinigungsmittel, die als Poliermittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalt von 15 bis 35 Gew,-%, so werden die sogenannten Hydrogelkieselsäuren erhalten, wie sie z.B. aus US 4,153,680 bekannt sind. Durch Trocknung auf Wassergehalte unterhalb 15 Gew.-% erfolgt eine irreversible Schrumpfung der vorher lockeren Struktur des Hydrogels zur dichten Struktur des sog. Xerogels. Solche Xerogelkieselsäuren sind z.B. in US 3,538,230 beschrieben.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Herstellung von Fällungskieselsäuren sind z.B. in DE-OS 25 22 486 und in DE-OS 31 14 493 beschrieben. Bevorzugt geeignet ist eine gemäß DE-OS 31 14 493 hergestellte Fällungskieselsäure mit einer BET-Oberfläche von 15 - 110 m²/g, einer Partikelgröße von 0,5 - 20 um, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident® 12 DS (DEGUSSA) erhältlich.

Andere Fällungskieselsäuren dieser Art sind Sident 8 (DEGUSSA) und Sorbosil AC 39 (Crosfield Chemicals). Diese Kieselsäuren zeichnen sich durch eine geringere Verdickungswirkung und eine etwas höhere mittlere Teilchengröße von 8―14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 - 100 Pas aufweisen.

Zahnpasten, die eine deutlich höhere Viskosität von mehr als 100 Pas (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, sogenannte Verdickungskieselsäuren mit einer BET-Oberfläche von 150 ―250 m²/g zu, z.B. die Handelsprodukte Sipernat 22 LS oder Sipernat 320 DS.

Als weitere Poliermittelkomponente kann auch z.B. Aluminiumoxid in Form von schwach calcinierter Tonerde mit einem Gehalt an γ- und α-Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Als Fluorverbindungen können die erfindungsgemäßen Zahnpflegemittel z.B. Natriumfluorid. Zinkfluorid, Zinn-(II)-fluorid, Aminfluorid oder Natriummonofluorophosphat enthalten. Bevorzugt sollte eine Menge von 0,01 - 0,2 Gew.-% Fluor in Form der genannten Verbindungen enthalten sein.

Als Feuchthaltemittel können Glycerin, Sorbit, Xylit, Propylenglycol, Polyethylenglycol oder Mischungen dieser Stoffe eingesetzt werden. Bevorzugt enthalten die erfindungsgemäßen Zahnpflegemittel als Feuchthaltemittel ein Gemisch aus Glycerin, Sorbit und Polyethylenglycol im Gewichtsverhältnis 10 : (8 - 12) : (0,1- 1).

Als Bindemittel und Konsistenzregler dienen z.B. natürliche und synthetische wasserlösliche Polymere wie z.B. Carragheen, Traganth, Guar, Cellulose und deren nichtionogene Derivate wie z.B. Hydroxyethylcellulose oder Methylhydroxypropylcellulose. Auch Agar-Agar, Xanthan-Gum, Pectine, wasserlösliche Carboxyvinylpolymere (z.B. Carbopol®-Typen), Polyvinylalkohol, Polyvinylpyrrolidon und höhermolekulare Polyethylenglycole (mit Molekulargewichten von 10³ - 10⁶ D) eignen sich als Binde- und Verdickungsmittel.

Als nicht-kationische, bakterizide Komponente eignen sich z.B. Phenole, Resorcine, Bisphenole, Salicylanilide und deren halogenierte Derivate, halogenierte Carbanilide und p-Hydroxybenzoesäureester. Besonders bevorzugte antimikrobielle Komponenten sind halogenierte Diphenylether, z.B. 2,4-Dichlor-2'-hydroxydiphenylether, 4,4'-Dichlor-2'hydroxydiphenylether, 2.4,4' -Tribrom-2'-hydroxydiphenylether und 2,4,4'-Trichlor-2'-hydroxydiphenylether (Triclosan). Sie werden bevorzugt in Mengen von 0,01 - 1 Gew.-% in die erfindungsgemäßen Zahnpflegemittel eingesetzt. Besonders bevorzugt wird Triclosan in einer Menge von 0,01 - 0,3 Gew.-% eingesetzt.

D-Panthenol [D - (+) - 2.4-Dihydroxy-N-(3-hydroxypropyl)-3,3-dimethyl-butyrarnid] zeigt eine der Pantothensäure entsprechende biologische Aktivität. Die Pantothensäure (R - (+) - N - (2.4-Dihydroxy-3,3-dimethylbutyryl-ß-alanin) ist eine Vorstufe in der Biosynthese des Coenzyms A und wird zum Vitamin-B-Komplex (B₃) gezählt. Diese Stoffe sind dafür bekannt, daß sie die Wundheilung fördern und eine günstige Wirkung auf die Haut haben. Sie sind daher auch gelegentlich in Zahnpasten beschrieben worden. Die erfindungsgemäßen Zahnpflegemittel enthalten bevorzugt 0,05 - 5 Gew.-% Panthenol oder ein Salz der Pantothensäure.

Retinol (3,7-Dimethyl-9-(2,6,6-trimethyl-1-cyclohexenyl)-2,4,6,8-nonatetraen-1-ol ist der internationale Freiname für Vitamin A₁. Anstelle des Retinols kann auch eines seiner Derivate mit ähnlicher biologischer Wirkung, z.B. ein Ester oder die Retinoesäure (Tretinoin), eines ihrer Salze oder ihre Ester verwendet werden. Bevorzugt wird ein Retinol-Ester, insbesondere ein Fettsäureester einer Fettsäure mit 12 - 22 C-Atomen verwendet. Besonders bevorzugt ist Retinol-Palmitat geeignet. Bei Verwendung eines Retinol-Esters, z.B. Retinol-Palmitat mit einer Aktivität von 1,7 10⁶ I.E. pro g ist eine Menge von 0,001 bis 0,1 Gew.-% bevorzugt. Bei Verwendung anderer Retinol-Derivate empfiehlt sich eine Einsatzmenge, die einer Konzentration von 10³ bis 10⁶ I.E. (Internationale Einheiten) pro 100 g entspricht.

Bevorzugte Zahnpflegemittel gemäß der vorliegenden Erfindung enthalten neben Poliermitteln, Fluorverbindungen, Feuchthaltemitteln und Bindemitteln bevorzugt
0,01 - 1Gew.-% eines halogenierten Diphenylethers
0,05 - 5 Gew.-% Panthenol oder ein Salz der Pantothensäure und
0,01 - 0,1 Gew.-% eines Retinol-Esters, bevorzugt Retinol-Palmitat.

Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung. da sie sowohl Calcium- als auch Phosphationen liefert. In den erfindungsgemäßen Zahnpflegemitteln wird Calciumglycerophosphat bevorzugt in Mengen von 0,01 - 1 Gew.-% eingesetzt. Insgesamt können die erfindungsgemäßen Zahnreinigungsmittel übliche Hilfsmittel und Zusatzstoffe in Mengen bis zu 10 Gew.-% enthalten.

Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose.

Weitere übliche Hilfs- und Zusatzstoffe für Zahnpasten sind
- Oberfkächenaktive Stoffe, bevorzugt anionische, zwitterionische, amphotere, nichtionische Tenside oder eine Kombination mehrerer verschiedener Tenside
- Lösungsmittel und Lösungsvermittler, z.B. niedere einwertige oder mehrwertige Alkohole oder Ether, z.B. Ethanol, 1,2-Propylenglycol, Diethylenglycol oder Butyldiglycol
- Pigmente, wie z.B. Titandioxid
- Farbstoffe
- Puffersubstanzen. z.B. primäre, sekundäre oder tertiäre Alkaliphosphate oder Citronensäure-/Na-Citrat
- weitere wundheilende oder entzündungshemmende Stoffe, z.B. Allantoin, Harnstoff, Azulen, Kamillewirkstoffe, Acetylsalicylsäurederivate oder Rhodanid
- weitere Vitamine wie z.B. Ascorbinsäure, Biotin, Tocopherol oder Rutin
- Mineralsalze wie z.B. Mangan-, Zink- oder Magnesiumsalze.

Die folgenden Beispiele sollen den Erfindungsgegenstand näher erläutern:

### Beispiele

### I. Erfindungsgemäße Zahnpasten wurden nach folgender Rezeptur hergestellt:

| | 1 | 2 |
|---|---|---|
| Wasser | ad 100 | ad 100 |
| Sorbit | 20,0 | 20,0 |
| Glycerin | 21,0 | 21,0 |
| Polyethylenglycol 1550 | 2,0 | 1,0 |
| Sident 8 SPLS | 12,0 | - |
| Sident 12 SPLS | - | 12,0 |
| Sident 22 LS | 8,0 | 5,5 |
| Sipernat 320 DS | 1,0 | 0,5 |
| TiO₂ | 1,0 | 1,0 |
| Na₂HPO₄ | 0,20 | 0,20 |
| Na₃PO₄ | 0,25 | 0,25 |
| Saccharin-Na | 0,20 | 0,20 |
| Na-Benzoat | - | 0,49 |
| Na-Fluorid | 0,32 | 0,32 |
| Phosphorsäure | - | 0,1 |
| Carboxymethylcellulose | 0,6 | - |
| Xanthan-Gum | - | 0,8 |
| Na-Laurylsulfat | 1,35 | 1,35 |
| MgSO₄·7H₂O | 0,210 | 0,210 |
| ZnSO₄·7H₂O | 0,088 | 0,088 |
| MnSO₄ ·H₂O | 0,011 | 0,011 |
| Farbstoffe | 0,55 | 0,0055 |
| Ca-Glycerophosphat | 0,130 | 0,130 |
| Triclosan | 0,100 | 0,100 |
| Vitamin-A₁-palmitat (1.7 Mio I.E./g) | 0,018 | 0,03 |
| D-Panthenol (50 %ig) | 0,50 | 0,25 |
| Aroma | 1,00 | 1,00 |

### II. Erfindungsgemäße flüssige Zahncremes

| | **3** | **4** | **5** |
|---|---|---|---|
| Wasser | ad 100 | ad 100 | ad 100 |
| Sorbit (70 %ig) | 30,0 | 30,0 | 30,0 |
| Glycerin (86 %ig) | 33,0 | 33,0 | 33,0 |
| Polyethylengiycol 1550 | 1,0 | 1,0 | 1,0 |
| Ethanol (93,8 %ig) | 2,0 | 2,0 | 2,0 |
| Sident 8 | 12,0 | 12,0 | - |
| Sident 12 SPLS | - | - | 12,0 |
| TiO₂ | 1,0 | 1,0 | - |
| Na₂HPO₄ | 0,2 | 0,2 | 0,2 |
| Saccharin-Na | 0,2 | 0,2 | 0,2 |
| Na-Benzoat | 0,49 | 0,49 | 0,5 |
| Na-Fluorid | 0,32 | 0,23 | 0,33 |
| Xanthan-Gum | 0,5 | 0,5 | 0,3 |
| Na-Laurylsulfat | 1,5 | 1,5 | 1,5 |
| PEG30-Glyceryl-Stearat | 0,5 | 0,5 | 0,5 |
| MgSO₄·7H₂O | 0,21 | 0,21 | 0,21 |
| ZnSO₄·7H₂O | 0,088 | 0,088 | 0,088 |
| MnSO₄·H₂O | 0,011 | 0,011 | 0,011 |
| Farbstoffe | 0,0055 | 0,0055 | 0,00067 |
| Ca-Glycerophosphat | 0,13 | 0,13 | |
| Tridosan | 0,1 | 0,1 | 0,3 |
| Vitamin A-palmitat (1,7 Mio I.E./g) | 0,018 | 0,01 | 0,025 |
| D-Panthenol (50 %ig) | 0,5 | 0,5 | 0,26 |
| Aroma | 1,0 | 1,0 | 1,0 |

### III. Klinischer Vergleich

Die flüssige Zahncreme gemäß Beispiel 3 (A) wurde mit einer entsprechenden Placebo-Zahncreme (B) verglichen, die anstelle der Komponenten Vitamin-A-palmitat und Panthenol Wasser enthielt.

Zwei Gruppen von je vierzig Versuchspersonen unterließen drei Tage lang jegliches Zähneputzen, um eine Gingivitis gemäß dem experimentellen Gingivitis-Testverfahren zu entwickeln.

Danach benutzte die eine Gruppe die Zahnpaste (A) (erfindungsgemäß) und die andere Gruppe die Zahnpaste (B) zweimal täglich in üblicher Weise 1 Minute lang. Am ersten Tag der Zahncreme-Anwendung und am 7. Tag wurden die folgenden Parameter an 12 Zähnen unter Verwendung von Spiegel und Sonde von geschulten Dentisten bestimmt.
- Der Gingival-Index (nach Silness-Löe)
   (Bewertung 0-3)
- Der Entzündungsgrad
   (Bewertung 0 - 3)
- Das spontane Schmerzempfinden.
   (Bewertung 0 - 3)

Zusätzlich wurde von den Versuchspersonen ein Fragebogen ausgefüllt, um die Akzeptanz der Produkte zu ermitteln.

### Ergebnisse:

Die Bewertungen sind als Mittelwert über alle Versuchspersonen (n = Anzahl der Versuchspersonen) angegeben. In Klammern ist die Standard-Abweichung angegeben.

### 1. Gingivitis-Index

| | **n** | **Zahncreme A** **(Beispiel 3)** | **n** | **Placebo-Creme (B)** |
|---|---|---|---|---|
| 1. Tag | 40 | 1,252 (0,519) | 40 | 1,199 (0,473) |
| 7. Tag | 40 | 0,116 (0,149) | 40 | 0,499* (0,385) |

| | | | | |
|---|---|---|---|---|
| * Die statistische Signifikanz (nach dem Kruskal Wallis Test) ist 99,9 %. | | | | |

Daraus errechnet sich eine Gingivitis-Reduktion von

| | |
|---|---|
| 1,136 | (Bewertungspunkten) für die erfindungsgemäße Zahncreme gegenüber nur |
| 0,7 | (Bewertungspunkten) für die Placebo-Zahncreme ohne Vitamin-A-palmitat und Panthenol |

### 2. Entzündungsgrad

| | **n** | **Zahncreme A** **(Beispiel 3)** | **n** | **Placebo-Creme (B)** |
|---|---|---|---|---|
| 1. Tag | 40 | 1.325 | 40 | 1,3 |
| 7. Tag | 40 | 0,30 | 40 | 0,525* |

| | | | | |
|---|---|---|---|---|
| * Statistische Signifikanz: 95 % | | | | |

### 3. Spontanes Schmerzempfinden

| | **n** | **Zahncreme A** **(Beispiel 3)** | **n** | **Placebo-Creme (B)** |
|---|---|---|---|---|
| 1. Tag | 40 | 0,65 | 40 | 0,57 |
| 7. Tag | 40 | 0,0 | 40 | 0,07 |

Das Ergebnis des klinischen Vergleichs zeigte deutlich, daß die erfindungsgemäße Zahncreme (A) (Beispiel 3) innerhalb der 1. Woche der Anwendung die Gingivitis und Entzündungen deutlicher mindert als die Placebo-Creme.

## Patentansprüche

1. Zahnpflegemittel mit entzündungshemmender Wirkung mit einem Gehalt an Poliermitteln, Fluorverbindungen, Feuchthaltemitteln, Bindemitteln, einer wasserunlöslichen, nicht-kationischen bakteriziden Komponente und Wasser, **dadurch gekennzeichnet, daß** zur Verbesserung der entzündungshemmenden Eigenschaften eine Kombination von
- Panthenol oder einem Salz der Pantothensäure und
- Retinol oder einem Derivat davon
enthalten ist.

2. Zahnpflegemittel gemäß Anspruch 1, **dadurch gekennzeichnet, daß** als bakterizide Komponente ein halogenierter Diphenylether enthalten ist.

3. Zahnpflegemittel gemäß einem der Ansprüche 1 oder 2, **dadurch gekennzeichnet, daß** eine Kombination aus
- 0,01- 1 Gew.-% eines halogenierten Diphenylethers
- 0.05 - 5 Gew.-% Panthenol oder einem Salz der Pantothensäure und
- 0,001 - 0,1 Gew.-% eines Retinol-Esters, bevorzugt Retinol-Palmitat
enthalten ist.

4. Zahnpflegemittel gemäß einem der Ansprüche 1 - 3, **dadurch gekennzeichnet, daß** zusätzlich 0,01 - 1 Gew.-% Calcium-Glycerophosphat enthalten ist.

5. Zahnpflegemittel nach einem der Ansprüche 1 - 3, **dadurch gekennzeichnet, daß** weitere Hilfs- und Zusatzstoffe in einer Menge von bis zu 10 Gew.-% enthalten sind.

## Claims

1. A dental care preparation with anti-inflammatory properties containing polishing components, fluorine compounds, humectants, binders, a water-insoluble non-cationic bactericidal component and water, **characterized in that** it contains a combination of
- panthenol or a salt of pantothenic acid and
- retinol or a retinol derivative
to improve it anti-inflammatory properties.

2. A dental care preparation as claimed in claim 1, **characterized in that** it contains a halogenated diphenylether as the bactericidal component.

3. A dental care preparation as claimed in claim 1 or 2, **characterized in that** it contains a combination of
0.01 to 1% by weight of a halogenated diphenylether,
0.05 to 5% by weight of panthenol or a salt of pantothenic acid and
0.001 to 0.1% by weight of a retinol ester, preferably retinol palmitate.

4. A dental care preparation as claimed in any of claims 1 to 3, **characterized in that** it additionally contains 0.01 to 1% by weight of calcium glycerophosphate.

5. A dental care preparation as claimed in any of,claims 1 to 3, **characterized in that** it contains other auxiliaries and additives in a quantity of up to 10% by weight.

## Revendications

1. Dentifrice ayant une action anti-inflammatoire avec une teneur en agents de polissage, en composées de fluor, en conservateurs d'humidité, en liants, en composants bactéricides non cationiques insolubles dans l'eau et en eau, **caractérisé en ce que**
pour l'amélioration des propriétés anti-inflammatoires, il contient une combinaison de :
- panthénol ou d'un sel de l'acide pantothénique, et
- de rétinol ou d'un dérivé de celui-ci.

2. Dentifrice conformément à la revendication 1,
**caractérisé en ce qu'**
il contient comme composant bactéricide un diphényléther halogéné.

3. Dentifrice conformément à l'une des revendications 1 ou 2,
**caractérisé en ce qu'**
il contient une combinaison à base de
- 0,01 à 1 % en poids d'un diphényléther halogéné,
- 0,05 à 5 % de panthénol ou d'un sel de l'acide pantothénique, et de 0,001 à 0,1 % en poids d'un ester de rétinol, de préférence le palmitate de rétinol.

4. Dentifrice conformément à l'une des revendications 1 à 3,
**caractérisé en ce qu'**
en supplément, il contient de 0,01 à 1 % en poids de glycérophosphate de calcium.

5. Dentifrice selon l'une des revendications 1 à 3,
**caractérisé en ce que**
d'autres adjuvants et additifs sont contenus en une quantité allant jusqu'à 10 % en poids.
